# NOUVEAU FASCICULE DE BREVET EUROPEEN

(11) **EP 2 018 848 B2**
(45) Date de publication et mention de la décision concernant l'opposition: **31.08.2016**
(45) Mention de la délivrance du brevet: 09.01.2013
(21) Numéro de dépôt: 08160739.2
(22) Date de dépôt: 18.07.2008
(51) Int. Cl.: A61K 8/19, A61Q 5/08, A61K 8/49, A61K 8/43, A61Q 5/04, A61Q 5/06, A61Q 5/10

(54) **Composition capillaire comprenant au moins un colorant fluorescent et au moins un agent alcalin hydroxyde, et procédé de mise en forme, de coloration et/ou d'éclaircissement simultanés.**
Haarzusammensetzung, die mindestens ein fluoreszierendes Färbemittel und mindestens eine Alkalihydroxidsubstanz enthält, und Verfahren zur gleichzeitigen Formgebung, Färbung und/oder Aufhellung
Hair composition comprising at least one fluorescent dye and at least one alkali hydroxide agent and method of simultaneous styling, dyeing and/or lightening.

(30) Priorité: 24.07.2007 FR 0756705
(43) Date de publication de la demande: 28.01.2009
(73) Titulaire: L'Oréal, 75008 Paris (FR)
(72) Inventeur: Guerin, Frédéric, 75010, PARIS (FR)
(74) Mandataire: Dossmann, Gérard

(56) Documents cités:
- EP-A- 0 226 860
- EP-A- 1 464 318
- EP-A- 1 464 326
- FR-A- 2 393 572
- GB-A- 759 385
- GB-A- 876 663
- US-A- 5 094 662
- DATABASE WPI Thomson Scientific, London, GB; AN 1990-128328 XP002472487 "Hair-treating agent compsn. for e.g. permanent colouring and waving-contains e.g. mercapto reductant alkali, viscosity adjuster e.g. water soluble polymer, alcohol and anionic agent, and colourants." & JP 02 076807 A (SAN-EI CHEM IND LTD) 16 mars 1990 (1990-03-16)

## Description

La présente invention a pour objet une composition capillaire de coloration et de mise en forme des cheveux comprenant un colorant fluorescent et un agent alcalin hydroxyde.

Il existe divers traitements appliqués aux fibres kératiniques humaines dans le but d'en changer l'aspect. A titre d'exemples de procédé permettant de changer la couleur, on peut citer la décoloration ou la coloration des fibres. A titre d'exemple de procédé permettant de modifier la forme des fibres, on peut citer le défrisage ou décrêpage ou lissage.

Pour ce qui concerne la coloration, on connaît deux types de procédés, la coloration d'oxydation et la coloration directe.

La coloration d'oxydation consiste à mettre en oeuvre, en présence d'un agent oxydant, des bases d'oxydation (telles que par exemple des ortho- ou para-phénylènediamines, des ortho- ou para-aminophénols et des composés hétérocycliques), éventuellement associées à des coupleurs (comme notamment les méta-diamines aromatiques, les méta-aminophénols, les méta-diphénols et certains composés hétérocycliques).

La coloration directe ou semi-permanente consiste à appliquer des colorants directs, qui sont des molécules colorées et colorantes ayant une affinité pour les fibres, à laisser poser pour permettre aux molécules colorées de pénétrer par diffusion à l'intérieur de la fibre, puis à les rincer. On peut utiliser par exemple des colorants directs nitrés benzéniques, anthraquinoniques, nitropyridiniques, azoïques, xanthéniques, acridiniques, aziniques ou triarylméthaniques.

Il est aussi possible de colorer les fibres kératiniques à partir de colorants disulfures. De tels colorants sont par exemple décrits dans le document FR 2876576.

La décoloration est obtenue par l'emploi d'un agent oxydant, qui, en fonction de sa nature, permet de décolorer la fibre ou simplement de l'éclaircir. Ainsi, en utilisant du peroxyde d'hydrogène ou l'un de ses précurseurs en milieu en général alcalin, l'éclaircissement de la fibre est plutôt léger, et en utilisant des composés du type des sels peroxygénés (persulfate, perborate de métaux alcalins ou d'ammonium), en association avec du peroxyde d'hydrogène en milieu alcalin, la fibre est plus fortement décolorée.

Il est aussi possible d'observer un éclaircissement optique des cheveux foncés par l'application de colorants fluorescents. Cet éclaircissement optique ne met pas en oeuvre d'agent oxydant. Un tel effet est décrit par exemple dans la demande de brevet EP 1432390.

La mise en forme du cheveu peut être réalisée selon plusieurs méthodes. Une de ces méthodes consiste à mettre en contact les fibres avec une composition très fortement alcaline, comprenant des hydroxydes, et qui a pour résultat de transformer des ponts disulfures par lanthionisation. Ce type de procédé est en général employé pour défriser des cheveux.

Si l'on ne rencontre pas de problème particulier en mettant en oeuvre l'un des traitements précités, de manière isolée, il n'en est pas de même lorsque l'on souhaite combiner une mise en forme du cheveu avec une modification de la couleur. En effet, les étapes de mise en forme permanente, et plus particulièrement de défrisage, sont des procédés fragilisant les fibres et il est souvent difficilement envisageable de mettre en oeuvre par la suite, de manière rapprochée, une étape de coloration et/ou d'éclaircissement, sans risquer une dégradation très importante de la fibre.

Il est connu du document US 2002/192175 un procédé de défrisage et de coloration à partir de colorants cationiques, d'un défrisant, d'un émulsifiant, d'un hydratant et d'un agent conditionneur. Les résultats coloriels obtenus ne sont pas satisfaisants et les colorants décrits ne permettent pas d'obtenir un éclaircissement simultané des cheveux foncés.

Le document EP-A-1 464 318 décrit un procédé de coloration avec effet éclaircissant de fibres kératiniques ayant subi une déformation permanente, qui consiste à appliquer sur des fibres mises en forme antérieurement une composition comprenant un colorant fluorescent soluble. Ce procédé nécessitant deux étapes est contraignant et consommateur de temps. Les résultats coloriels ne sont pas toujours satisfaisants, notamment en terme d'intensité.

Le but de la présente invention est de pouvoir mettre en forme et colorer simultanément les fibres kératiniques, notamment pour obtenir un éclaircissement des cheveux foncés, tout en limitant la dégradation des fibres kératiniques.

Ce but est atteint par la présente invention qui a pour objet une composition de traitement des fibres kératiniques, notamment pour la mise en forme, la coloration et/ou l'éclaircissement simultanés, comprenant dans un milieu cosmétiquement acceptable,
* un ou plusieurs colorants fluorescents, tels que définis ci-après, solubles dans ledit milieu,
* un ou plusieurs agents alcalins de type hydroxyde minéral ou organique en quantités telles que le pH de la composition est compris entre 10 et 14, et
* un ou plusieurs tensioactifs non ioniques, anioniques, cationiques ou amphotères.

L'invention a de même pour objet un procédé de mise en forme et de coloration et/ou d'éclaircissement simultanés des fibres kératiniques, dans lequel on applique la composition tinctoriale comprenant dans un milieu cosmétiquement acceptable, un ou plusieurs colorants fluorescents, tels que définis ci-après, solubles dans ledit milieu, un ou plusieurs agents alcalins de type hydroxyde minéral ou organique en quantités telles que le pH de la composition est compris entre 10 et 14, pendant un temps suffisant pour développer la mise en forme, la coloration et/ou l'éclaircissement souhaités, puis on rince éventuellement les fibres kératiniques et on les lave au shampooing.

L'invention concerne aussi un dispositif à plusieurs compartiments comportant un premier compartiment comprenant, dans un milieu cosmétiquement acceptable, un ou plusieurs agents alcalins de type hydroxyde minéral ou organique et un deuxième compartiment comprenant un ou plusieurs colorants fluorescents tels que définis ci-après, le pH du mélange du contenu des différents compartiments étant compris entre 10 et 14.

L'invention concerne également un dispositif à plusieurs compartiments comportant un premier compartiment comprenant du carbonate de guanidine et un ou plusieurs colorants fluorescents, tels que définis ci-après, et un deuxième compartiment comprenant un ou plusieurs agents alcalins de type hydroxyde d'un métal alcalin ou alcalino-terreux, le pH du mélange du contenu des différents compartiments étant compris entre 10 et 14.

L'invention concerne enfin un dispositif à plusieurs compartiments comportant un premier compartiment comprenant du carbonate de guanidine, un deuxième compartiment comprenant un ou plusieurs agents alcalins de type hydroxyde d'un métal alcalin ou alcalino-terreux, et un troisième compartiment comprenant un ou plusieurs colorants fluorescents tels que définis ci-après, le pH du mélange du contenu des différents compartiments étant compris entre 10 et 14.

La composition selon l'invention permet d'obtenir simultanément une mise en forme du cheveu, notamment un défrisage et une coloration sans dégradation supplémentaire des fibres kératiniques. En particulier, lorsque ce procédé est mis en oeuvre sur cheveux foncés, on peut observer un éclaircissement des cheveux sans avoir à mettre en oeuvre un agent oxydant. Mais bien entendu, il n'est pas exclu que la composition selon l'invention comprenne un tel agent.

Il est ainsi possible d'effectuer une mise en forme des cheveux, telle qu'un défrisage, une coloration ou un éclaircissement simultanés en utilisant une unique composition s'appliquant en une seule étape.

Par ailleurs, il a été également constaté que la présence de l'agent alcalin à pH élevé permettait une meilleure montée du colorant sur la fibre kératinique et un meilleur unisson, notamment en raison du gonflement des fibres dû audit agent alcalin.

Selon un mode de réalisation avantageux de l'invention, la composition est destinée à être appliquée sur des fibres kératiniques foncées. Plus particulièrement, les fibres kératiniques foncées sont des fibres pigmentées ou colorées artificiellement, dont la hauteur de ton est inférieure ou égale à 6 et de préférence inférieure ou égale à 4.

Il est rappelé que la notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage « Sciences des traitements capillaires » de Charles ZVIAK 1988, Ed.Masson, pp.215 et 278. Les hauteurs de ton s'échelonnent de 1 (noir) à 10 (blond clair clair), une unité correspondant à un ton ; plus le chiffre est élevé et plus la nuance est claire.

Il est à noter que la composition est appropriée pour le traitement de fibres kératiniques, quelle que soit leur coloration avant traitement et que celle-ci soit naturelle ou obtenue artificiellement.

Il est à noter que dans ce qui va suivre, et à moins d'une autre indication, les bornes d'un domaine de valeurs sont comprises dans ce domaine.

Par colorant fluorescent, on entend au sens de la présente invention un colorant qui est une molécule qui colore par elle-même, et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les ultraviolets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Un colorant fluorescent est différent d'un pigment fluorescent qui lui, n'est pas soluble dans le milieu de la composition.

Plus particulièrement, un colorant fluorescent dans le cadre de la présente invention, éventuellement neutralisé, est soluble dans le milieu de la composition à au moins 0,001 g/l, plus particulièrement au moins 0,5 g/l, de préférence au moins 1 g/l et, selon un mode de réalisation encore plus préféré, au moins 5 g/l à une température comprise entre 15 et 25°C.

Les colorants fluorescents utilisés de préférence selon la présente invention peuvent donner des couleurs dans la gamme des jaunes, orangés, violets, rouges ou verts.

De préférence, les colorants fluorescents de la composition selon l'invention présentent un maximum de réflectance se situant dans la gamme de longueurs d'onde allant de 500 à 650 nanomètres, et de préférence dans la gamme de longueurs d'onde allant de 550 à 620 nanomètres.

Les colorants fluorescents selon la présente invention sont des composés connus en eux-mêmes.

Les colorants fluorescents selon la présente invention sont des colorants fluorescents exempts de liaison disulfure.

Les colorants fluorescents mis en oeuvre, appartiennent à la famille suivante : les stilbazoliums.

On peut notamment citer parmi eux :
- les composés de structure suivante : 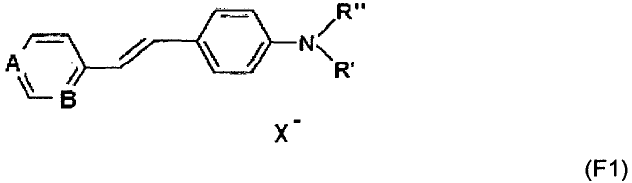
   formule dans laquelle A et B désignent un groupement CH ou un groupement N⁺R, un seul de A ou B désignant un groupement CH ; R représente un radical alkyle linéaire, ramifié ou cyclique comprenant 1 à 22 atomes de carbone, éventuellement substitué par au moins un groupement hydroxyle, par au moins un groupement alcoxy en C₁-C₆, linéaire ou ramifié, ou cycloalcoxy en C₁-C₆ et/ou par au moins un groupement cyano ;
   R', R" indépendamment l'un de l'autre, représentent un atome d'hydrogène ; un radical alkyle, linéaire ou ramifié, comprenant 1 à 22 atomes de carbone, éventuellement substitué par un ou plusieurs radicaux hydroxyles ;
   X représente un anion organique ou minéral.

Plus particulièrement, le radical R peut être un radical méthyle ou éthyle, et les radicaux R', R" indépendamment l'un de l'autre peuvent être un atome d'hydrogène ou un radical méthyle.

A titre d'exemple de composé de ce type on peut citer le 2-(2-(4-diméthylamino-phényl)-vinyl)-1-éthyl-pyridinium éthyl sulfate et le 4-(2-(4-diméthylaminophényl)-vinyl)-1 méthyl-pyridinum paratoluène sulfonate.

On peut également utiliser comme colorants fluorescents, les colorants de type méthines ou cyanines choisis parmi les composés de formules (F9) ou (F10) suivantes et leurs sels d'addition avec un acide ou une base : 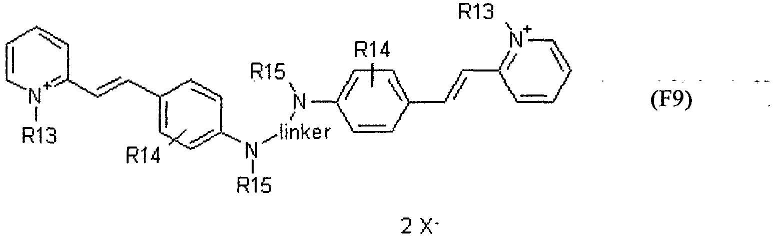 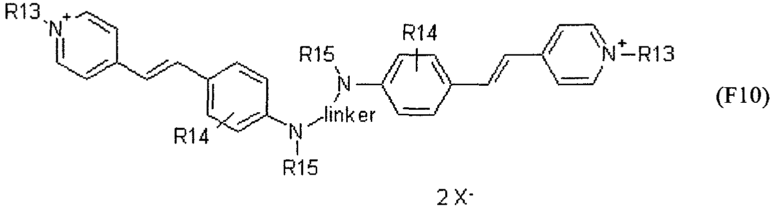 dans lesquelles :
- Linker représente une chaîne hydrocarbonée en C₁-C₁₂, de préférence en C₂-C₈, aliphatique ou alicyclique, saturée ou insaturée, un ou plusieurs atomes de carbone de la chaîne hydrocarbonée pouvant être remplacés par un ou plusieurs atomes d'oxygène, un ou plusieurs groupements NR avec R étant un atome d'hydrogène ou un radical alkyle, la chaîne hydrocarbonée ne comportant pas de groupement diazo, nitro, nitroso ou peroxyde, et la chaîne hydrocarbonée ne pouvant pas être terminée à l'une ou l'autre de ses extrémités par un hétéroatome ;
- R₁₃ représente un radical alkyle en C₁-C₈, de préférence en C₁-C₄, linéaire, éventuellement substitué en position terminale par un radical hydroxyle ou un radical alcoxy ;
- R₁₄ représente un atome d'hydrogène ; un radical halogéno ; un radical alkyle ; un radical alcoxy ; un radical amino éventuellement substitué par un ou plusieurs radicaux alkyle en C₁-C₄ eux-même éventuellement substitués par un ou plusieurs radicaux hydroxyle ;
- R₁₅ représente un radical alkyle en C₁-C₈, de préférence en C₁-C₄, linéaire, éventuellement substitué en position terminale par un radical hydroxyle ou un radical alcoxy ;
   les deux radicaux R₁₅ pouvant former avec le linker et les atomes d'azote auxquels ils sont reliés un hétérocycle saturé à 6 ou 7 chaînons de formule suivante : 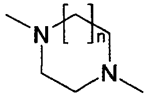 avec n égal à 1 ou 2 ;
- X⁻ représente un contre-ion.

Le linker peut être choisi parmi un radical alkylène ; un radical alkylène aralkylène. De préférence, le linker est choisi parmi un radical éthylène ; un radical propylène ; un radical butylène ; un radical hexylène ; un groupement de formule suivante :

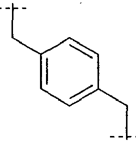

Encore plus préférentiellement, le linker peut être choisi parmi un radical éthylène ; un radical butylène ; un radical hexylène.

Selon une variante, R₁₃ représente un radical alkyle. De préférence, R₁₃ est choisi parmi un radical méthyle ; un radical éthyle.

Selon une autre variante, R₁₄ est choisi parmi un atome d'hydrogène ; un radical alkyle ; un radical alcoxy ; un radical halogéno. De préférence, R₁₄ est un atome d'hydrogène.

Selon une autre variante, R₁₅ représente un radical alkyle. De préférence, R₁₅ représente un radical méthyle.

A titre d'exemples de contre-ions X⁻, on peut citer les ions halogénure tels que l'ion chlorure, bromure, fluorure ou iodure, l'ion hydroxyde, l'ion hydrogènesulfate, les ions alkylsulfate en C₁-C₆ tels que le méthylesulfate ou l'éthylsulfate.

A titre d'exemples de composés de formules (F9) ou (F10), on peut citer les composés présentés dans le tableau ci-dessous :

| |
|---|
| 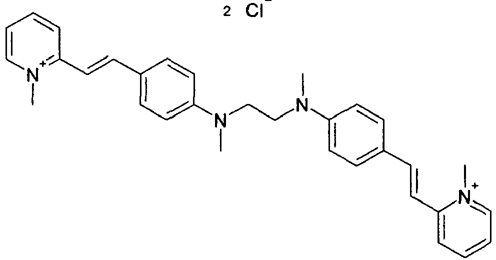 |
| dichlorure de 1-méthyl-2-[(E)-2-(4-{méthyl[2-(méthyl{4-[(E)-2-(1-méthylpyridinium-2-yl)vinyl]phényl}amino)éthyl]amino} phényl)vinyl]pyridinium |
| 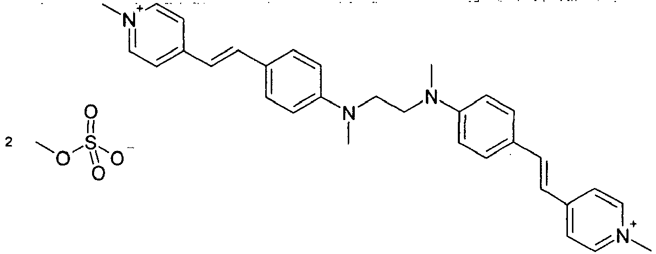 |
| bis(méthylsulfate) de 1-méthyl-4-[(E)-2-(4-{méthyl[2-(méthyl{4-[(E)-2-(1-méthylpyridinium-4-yl)vinyl]phényl}amino)éthyl]amino} phényl)vinyl]pyridinium |
| 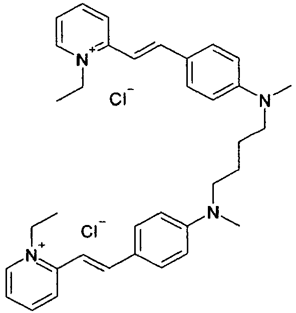 |
| dichlorure de 1-éthyl-2-((E)-2-{4-[{4-[{4-[(E)-2-(1-éthylpyridinium-2-yl)vinyl]phényl}(méthyl)amino]butyl}(méthyl)amino]phényl} vinyl)pyridinium |
| 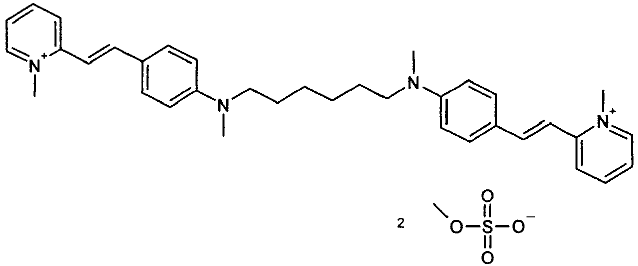 |
| bis(méthylsulfate) de 1-méthyl-2-[(E)-2-(4-{méthyl[2-(méthyl{4-[(E)-2-(1-méthylpyridinium-2-yl)vinyl]phényl}amino)hexyl]amino} phényl)vinyl]pyridinium |
| 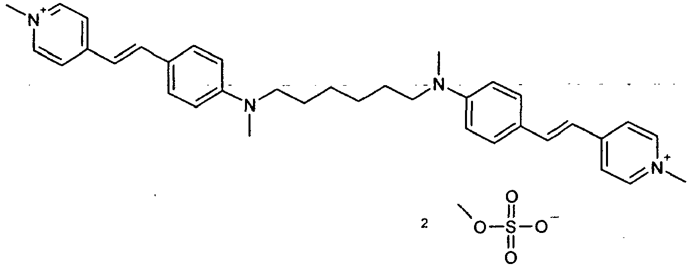 |
| bis(méthylsulfate) de 1-méthyl-4-[(E)-2-(4-{méthyl[2-(méthyl{4-[(E)-2-(1-méthylpyridinium-4-yl)vinyl]phényl}amino)hexyl]amino} phényl)vinyl]pyridinium |

Les composés de formules (F9) ou (F10) peuvent par exemple être préparés selon les modes de synthèse tels que décrits dans le brevet US 4 323 362. Ils peuvent en particulier être synthétisés selon l'un des modes opératoires suivants :

### Voie 1 :

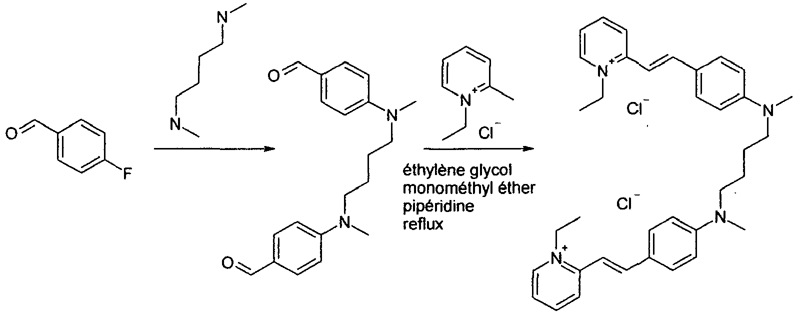

La substitution nucléophile d'un radical fluoro sur un système aromatique, et en particulier sur le 4-fluorobenzaldéhyde, est décrite dans la littérature. On peut notamment citer les références suivantes : Synthesis (8), 606-8, 1981 et Helvetica Chimica Acta 68(3), 584-91, 1985. Des alternatives existent pour cette première étape, par exemple celle qui est décrite dans la référence JOC Section C - Organic, 7, 1966, 666-8. L'étape de condensation est décrite dans la référence suivante : J. Heterocyclic Chemistry 16(8), 1583-7, 1979.

### Voie 2 :

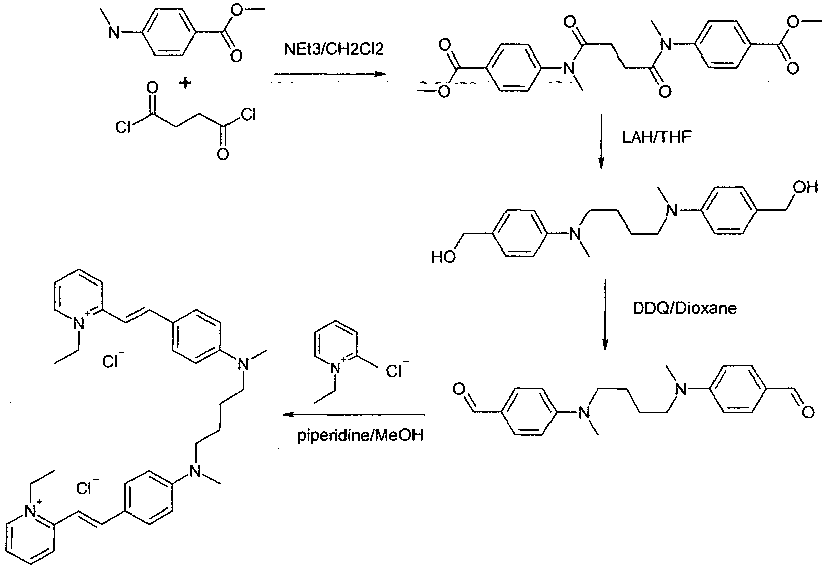

Les deux premières étapes de synthèse s'inspirent des conditions décrites dans la référence suivante : Farmaco 1989,1167. La troisième étape de synthèse s'inspire des conditions décrites dans la référence suivante : J.Chem.Soc. Perkin Trans I, 2000, 3559. L'étape de condensation est décrite dans la référence suivante : J. Heterocyclic Chemistry 16(8), 1583-7, 1979.

### Voie 3 :

Une voie de synthèse particulièrement intéressante est la suivante :

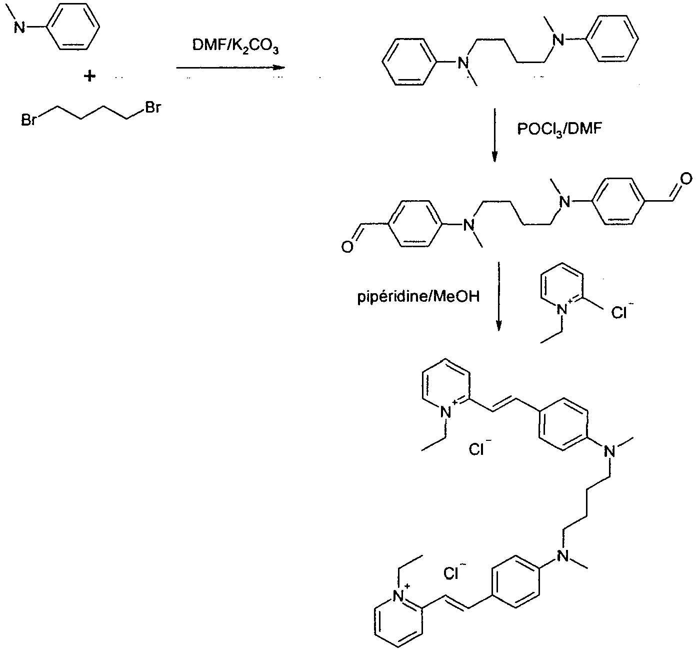

La réaction de N-alkylation d'une N-alkyl aniline et la réaction de formylation d'une N,N-dialkyle aniline sont respectivement décrites dans les références suivantes : Recueil des travaux chimiques des Pays-Bas et de la Belgique, 77, 559-568, 1958 et Organic préparations and procédures international, 36(4), 337-340, 2004. L'étape de condensation est décrite dans la référence suivante : J. Heterocyclic Chemistry 16(8), 1583-7, 1979.
- on peut citer les trimères et tétramères de stilbazolium suivants :

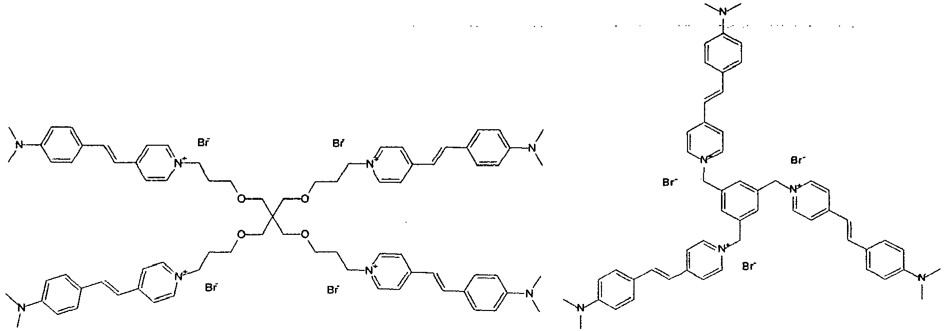

La teneur en colorant(s) fluorescent(s) selon la présente invention est notamment comprise entre 0,001 à 10% en poids, de préférence comprise entre 0,005 à 5% en poids, par rapport au poids total de la composition.

La composition selon l'invention comprend en outre un ou plusieurs agents alcalins de type hydroxyde minéral ou organique en quantité telle que le pH de la composition est compris entre 10 et 14, de préférence entre 12 et 14.

Plus particulièrement, l'agent alcalin de type hydroxyde est choisi parmi les hydroxydes de métaux alcalins ou alcalino-terreux, de métaux de transition, en particulier des groupes IIIB, IVB, VB et VIB, de lanthanides ou d'actinides, les hydroxydes d'ammonium, l'hydroxyde de guanidine, ou leurs mélanges.

A titre d'exemples de tels composés, on peut citer l'hydroxyde de sodium, l'hydroxyde de potassium, l'hydroxyde de lithium, l'hydroxyde de rubidium, l'hydroxyde de césium, l'hydroxyde de francium, l'hydroxyde de magnésium, l'hydroxyde de calcium, l'hydroxyde de strontium, l'hydroxyde de baryum, l'hydroxyde de manganèse, l'hydroxyde de zinc, l'hydroxyde de cérium, l'hydroxyde de lanthane, l'hydroxyde d'actinium, l'hydroxyde de thorium, l'hydroxyde d'aluminium, l'hydroxyde de guanidine et les hydroxydes d'ammonium quaternaires.

Il est à noter que certains hydroxydes, et plus particulièrement l'hydroxyde de guanidine, peuvent se trouver sous la forme de précurseurs, c'est-à-dire d'au moins deux composés qui mis en présence, conduisent par une réaction chimique, à l'hydroxyde de guanidine. A titre d'exemple, on peut donc citer l'association d'un hydroxyde de métal alcalino-terreux, comme par exemple le calcium, avec le carbonate de guanidine.

De préférence, on met en oeuvre, comme agent alcalin, l'hydroxyde de guanidine ou l'hydroxyde de sodium.

Comme indiqué précédemment, la quantité d'agent alcalin de type hydroxyde dans la composition est telle que le pH de la composition est d'au moins 10, plus particulièrement compris entre 12 et 14.

Avantageusement, la quantité d'agent alcalin de type hydroxyde est comprise entre 0,5 et 10 % en poids, et de préférence entre 1 et 8 % en poids par rapport au poids de la composition.

Le milieu cosmétiquement acceptable est de préférence un milieu comprenant de l'eau et éventuellement un ou plusieurs solvants organiques. Il est à noter que ces solvants peuvent être utilisés pour solubiliser des ingrédients qui ne le seraient pas suffisamment dans l'eau.

Parmi les solvants convenables, on peut citer les alcanols inférieurs en C₁-C₄, les polyols et éthers de polyols, les alcools aromatiques et leurs mélanges.

On peut citer plus particulièrement, les alcools tels que l'alcool éthylique, l'alcool isopropylique, l'alcool benzylique, et l'alcool phényléthylique, ou les polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore d'autres polyols comme le glycérol. On peut également utiliser comme solvants les polyéthylèneglycols et les polypropylèneglycols, et les mélanges de tous ces composés. On peut aussi utiliser le carbonate de propylène.

Le ou les solvants s'ils sont présents, représentent de préférence 1 à 40 % en poids par rapport au poids de la composition, et de préférence 5 à 30 % en poids.

Selon un mode de réalisation particulier de l'invention, la composition peut, comprendre, en plus du ou des colorants fluorescents tels que définis ci-avant, un ou plusieurs colorants directs additionnels non fluorescents de nature non ionique, cationique ou anionique.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

Les tensioactifs utilisés comprennent de préférence une chaîne alkyle ou acyle en C₁₀-C₂₄, plus particulièrement en C₁₂-C₂₄, et de préférence en C₁₂-C₂₂, éventuellement mono- ou poly- oxyalkylénée ou mono- ou poly-glycérolée.

En ce qui concerne les tensioactifs anioniques, on utilise habituellement les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants, seuls ou en mélange :
- les alkylsulfates, les alkyléthersulfates, les alkylamidoéthersulfates, les alkylarylpolyéthersulfates ;
- les alkylsulfonates, les alkylamidesulfonates, les alkyl-arylsulfonates ;
- les alkylsulfosuccinates, les alkyléthersulfosuccinates, les alkylamide-sulfosuccinates ;
- les alkylsulfoacétates ;
- les acylsarconisates ; et les acylglutamates :
- les esters d'alkyle et d'acides polyglycoside-carboxyliques tels que les glucoside-citrates d'alkyle, les polyglycoside-tartrates d'alkyle, et les polyglycoside-sulfosuccinates d'alkyle ;
- les alkylsulfosuccinamates ;
- les acyliséthionates, les N-acyltaurates ; les acyllactylates ;
- les acides d'alkyl-D-galactoside uroniques ;
- les acides alkyléther-carboxyliques polyoxyalkylénés, les acides alkylaryléther-carboxyliques polyoxyalkylénés, les acides alkylamidoéther carboxyliques polyoxyalkylénés ;
le groupe alkyle ou acyle (RCO-) de ces composés comportant de 10 à 24 atomes de carbone et le groupe aryle désignant de préférence un groupe phényle ou benzyle ; le nombre de groupements oxyalkylénés, et de préférence oxyéthylénés, est compris entre 2 et 50.

Pour ce qui concerne les tensioactifs non ioniques, ces derniers peuvent être avantageusement choisis parmi les composés suivants, seuls ou en mélange :
- les alcools gras polyéthoxylés, polypropoxylés ou polyglycérolés,
- les alpha-diols polyéthoxylés, polypropoxylés ou polyglycérolés,
   le nombre de groupements oxyde d'éthylène ou oxyde de propylène allant de 2 à 50 ; le nombre de groupements glycérol allant de 2 à 30 ;
- les copolymères d'oxyde d'éthylène et d'oxyde de propylène,
- les condensats d'oxyde d'éthylène et d'oxyde de propylène sur des alcools gras ;
- les alcanolamides gras polyéthoxylés ayant de 2 à 30 moles d'oxyde d'éthylène ;
- les amides gras polyglycérolés comportant de 1 à 5 groupements glycérol ;
- les esters éthoxylés d'acides gras du sorbitane ayant de 2 à 30 moles d'oxyde d'éthylène, les esters d'acides gras du saccharose, les esters d'acide gras du polyéthylèneglycol ;
- les alkylpolyglucosides, les dérivés de N-alkylglucamine ;
ces composés comprenant une chaîne alkyle ou acyle comprenant 10 à 24 atomes de carbone.

Les tensioactifs cationiques entrant dans la composition selon l'invention, peuvent notamment être choisis parmi les composés suivants, seuls ou en mélange :
- les sels d'amines grasses primaires, secondaires ou tertiaires, éventuellement polyoxyalkylénées,
- les sels d'ammonium quaternaire tels que les chlorures ou les bromures de tétraalkylammonium, d'alkylamidoalkyltrialkylammonium, de trialkylbenzyl-ammonium, de trialkylhydroxyalkylammonium, d'alkylpyridinium,
- les dérivés d'alkyl-imidazoline ;
ces composés comprenant au moins une chaîne alkyle comprenant 10 à 24 atomes de carbone.

Enfin, les tensioactifs amphotères peuvent être choisis parmi les composés suivants, seuls ou en mélange :
- les dérivés d'amines aliphatiques secondaires ou tertiaires, dans lesquels le groupe aliphatique est une chaîne linéaire ou ramifiée comportant 10 à 24 atomes de carbone et comprenant au moins un groupe anionique hydrosolubilisant tel que, par exemple, un groupe carboxylate, sulfonate, sulfate, phosphate ou phosphonate,
- les alkylbétaïnes, les sulfobétaïnes, les alkylamidoalkyl(C₆-C₈)bétaïnes, les alkylamidoalkyl(C₆-C₈)sulfobétaïnes ;
ces composés comprenant une chaîne alkyle comprenant de 10 à 24 atomes de carbone.

Parmi les tensioactifs, on préfère utiliser les alkylsulfates, les alkylbenzènesulfates, les alkyléthersulfates, les alkylsulfonates, les sels d'ammonium quaternaire, les alkylbétaïnes, les alcanolamides d'acides gras, les esters d'acides gras oxyéthylénés seuls ou en mélanges.

Le ou les tensioactifs sont généralement présents à une concentration de 0,5 à 30 % en poids, et de préférence, comprise entre 0,5 et 10 % en poids par rapport au poids total de la composition.

Dans le but d'améliorer les propriétés cosmétiques des fibres kératiniques traitées ou encore d'en atténuer ou d'en éviter la dégradation, la composition selon l'invention peut également comprendre un ou plusieurs agents traitants de nature cationique, anionique, non ionique ou amphotère.

On peut notamment utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2.535.730, les polyorganosiloxanes à groupements aminoalkyles modifiés par des groupements alcoxycarbonyalkyles tels que ceux décrits dans le brevet US n° 4.749.732, des polyorganosiloxanes tels que le copolymère polydiméthylsiloxane-polyoxyalkyle du type Diméthicone Copolyol, un polydiméthylsiloxane à groupements terminaux stéaroxy (stéaroxydiméthicone), un copolymère polydiméthylsiloxane-dialkylammonium acétate ou un copolymère polydiméthyl-siloxane polyalkylbétaïne décrits dans le brevet britannique n° 2.197.352, des polysiloxanes organo modifiés par des groupements mercapto ou mercaptoalkyles tels que ceux décrits dans le brevet français n° 1.530.369 et dans la demande de brevet européen n° 295.780, ainsi que des silanes tels que le stéaroxytriméthylsilane.

Les compositions selon l'invention peuvent également contenir d'autres ingrédients traitants tels que des polymères cationiques tels que ceux utilisés dans les compositions des brevets français n° 79.32078 (2.472.382) et 80.26421 (2.495.931), ou encore des polymères cationiques du type ionène tels que ceux utilisés dans les compositions du brevet luxembourgeois n°83703, des aminoacides basiques (tels que la lysine, l'arginine) ou acides (tels que l'acide glutamique, l'acide aspartique), des peptides et leurs dérivés, des hydrolysats de protéines, des cires dont les céramides, des alcools gras, des dérivés de la lanoline.

Les compositions selon l'invention se présentent essentiellement sous forme d'une crème épaissie de façon à maintenir les cheveux aussi raides que possible. On réalise ces crèmes, sous forme d'émulsions "lourdes", par exemple à base d'une phase liquide inerte.

A titre d'exemple de phase liquide inerte, on peut citer les polydécènes de formule C₁₀ₙH_{[(20n)+2]} dans laquelle n varie de 3 à 9 et de préférence de 3 à 7, les esters et en particulier les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales, ou leurs mélanges.

Les composés de formule C₁₀ₙH_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo^{®} 366 NF Polydecene par la société Amoco Chemical, ceux vendus sous la dénomination Nexbase^{®} 2002 FG, 2004 FG, 2006 FG et 2008 FG par la société Fortum.

En ce qui concerne les esters, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle et le stéarate d'octyl dodécyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de di-isopropyle,
- les esters de monoalcools linéaires ou ramifiés en C₃-C₈, avec des acides gras bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme phase liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme phase liquide inerte organique, comme par exemple l'huile de paraffine.

Les huiles végétales peuvent aussi convenir, et notamment l'huile d'avocat, l'huile d'olive ou la cire liquide de jojoba.

De préférence, la ou les phases liquides inertes organiques sont choisies parmi les polydécènes de formule C₁₀ₙH_{[(20n)+2]} dans laquelle n varie de 3 à 9 et de préférence de 3 à 7, les esters d'alcools gras ou d'acides gras, et leurs mélanges.

La concentration en phase liquide inerte organique représente de 5 à 60% en poids total de la composition.

On peut également utiliser des liquides ou des gels contenant des agents épaississants tels que des polymères ou des copolymères carboxyvinyliques qui "collent" les cheveux et les maintiennent dans la position lisse pendant le temps de pose.

Les compositions selon l'invention peuvent contenir, en outre, un ou plusieurs adjuvants choisis parmi les vitamines et pro-vitamines dont le panthénol, les filtres solaires hydrosolubles et liposolubles, siliconés ou non siliconés, les agents nacrants et opacifiants, les agents séquestrants, les agents plastifiants, les agents filmogènes, les céramides, les agents stabilisants, les agents de conditionnement, les agents épaississants minéraux et organiques différents des (co)polymères carboxyvinyliques, les agents antioxydants, les agents de pénétration, les parfums et les agents conservateurs.

Les adjuvants ci dessus sont en général présents en quantité comprise pour chacun d'eux entre 0,01 et 20 % en poids par rapport au poids de la composition.

L'invention concerne également un dispositif comprenant au moins deux compartiments, l'un des compartiments (i) comprenant, dans un milieu cosmétiquement acceptable, un ou plusieurs agents alcalins de type hydroxyde minéral ou organique, un autre compartiment (ii) comprenant, dans un milieu cosmétiquement acceptable, un ou plusieurs colorants fluorescents tels que définis ci-avant, la quantité d'agent alcalin de type hydroxyde étant telle que le pH après mélange du contenu des différents compartiments soit compris entre 10 et 14.

Il est à noter que dans le cas particulier d'un agent alcalin de type hydroxyde mis en oeuvre sous forme d'un précurseur, les composants précurseurs ne sont pas stockés dans le même compartiment. Ainsi, plus particulièrement dans le cas de la guanidine, le carbonate de guanidine est avantageusement stocké avec le colorant fluorescent et l'hydroxyde de métal alcalin ou alcalino-terreux dans un deuxième compartiment. On peut aussi avoir trois compartiments, le premier contenant le carbonate de guanidine, le second l'hydroxyde de métal alcalin ou alcalino-terreux et le troisième le colorant fluorescent.

Selon une première variante, le dispositif selon l'invention, comprend, en outre, une composition supplémentaire (iii) comprenant un ou plusieurs agents traitants et/ou un ou plusieurs adjuvants, tels que décrits précédemment.

Selon une deuxième variante, le ou les agents traitant et/ou le ou les adjuvants précités sont présents dans l'un et/ou l'autre des compartiments (i) ou (ii) et de préférence dans le compartiment (ii).

Les compositions de mise en forme, de coloration et/ou d'éclaircissement peuvent se présenter sous différentes formes, telles qu'une lotion, une crème, un gel ou sous toute autre forme appropriée pour réaliser une teinture des fibres kératiniques.

Les compositions du dispositif selon l'invention sont conditionnées dans des compartiments ou récipients ou dispositifs distincts, accompagnés, éventuellement, de moyens d'application appropriés, identiques ou différents, tels que les pinceaux, les brosses, les éponges. Elles peuvent également être conditionnées sous pression en flacon aérosol en présence d'un agent propulseur et former une mousse.

Un autre objet de l'invention concerne un procédé de mise en forme telle que le défrisage (ou lissage ou décrêpage) des fibres kératiniques mettant en oeuvre une composition cosmétique comprenant, dans un milieu cosmétiquement acceptable, un ou plusieurs colorants fluorescents précités et un ou plusieurs agents alcalins de type hydroxyde organique ou minéral tel que défini précédemment, lesdits agents étant présents dans la composition en quantité telle que le pH de la composition est compris entre 10 et 14, avantageusement entre 12 et 14.

Selon le procédé de mise en forme, de coloration et/ou d'éclaircissement simultanés des fibres kératiniques, on applique la composition tinctoriale sur les fibres kératiniques pendant un temps suffisant pour développer la mise en forme, la coloration et/ou l'éclaircissement souhaités. On rince ensuite éventuellement les fibres kératiniques et on les lave au shampooing.

Avantageusement, dans le procédé selon l'invention, la durée d'application est inférieure à 40 minutes, et plus avantageusement inférieure à 30 minutes

Selon un mode de réalisation préféré du procédé selon l'invention, on applique sur lesdites fibres la composition définie ci-avant, puis l'on soumet les cheveux à une déformation mécanique permettant de leur conférer une nouvelle forme, notamment par une opération de lissage des cheveux avec un peigne à larges dents, avec le dos d'un peigne ou à la main. Après un temps de pose, plus particulièrement de 5 à 60 minutes, de préférence, de 5 à 40 minutes, on procède alors éventuellement à un nouveau lissage, puis on rince les cheveux.

La composition est avantageusement appliquée sur des cheveux présentant une hauteur de ton inférieure ou égale à 6 et de préférence inférieure ou égale à 4.

La température d'application est généralement comprise entre la température ambiante (15 à 25°C) et 80°C et plus particulièrement entre 15 et 40°C. Ainsi, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de lissage de la chevelure, un fer chauffant à une température comprise entre 60 et 220°C et de préférence entre 120 et 200°C.

Lorsque la composition selon l'invention est utilisée pour traiter des fibres kératiniques foncées, telles que des cheveux châtains par exemple, la réflectance mesurée avant et après application permet d'évaluer l'éclaircissement obtenu. Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueurs d'onde allant de 500 à 700 nanomètres, est supérieure à celle correspondant aux cheveux non traités.

Cela signifie que, dans la gamme de longueurs d'onde allant de 500 à 700 nanomètres, et de préférence de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%.

Il est précisé toutefois qu'il peut exister dans la gamme de longueurs d'onde allant de 500 à 700 nanomètres, et de préférence de 540 à 700 nanomètres, une ou plusieurs plages où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Les exemples suivants servent à illustrer l'invention sans toutefois présenter un caractère limitatif.

### EXEMPLES DE MISE EN OEUVRE:

### EXEMPLE 1

On a préparé les compositions suivantes (teneurs exprimées en grammes de matière active) :

**Composition (i)**

| | |
|---|---|
| Propylène glycol | 50,5g |
| Silice | 3g |
| Hydroxyde de calcium | 45g |
| Dioxyde de titane | 1,5g |

**Composition (ii)**

| | |
|---|---|
| Colorant fluorescent (*) | 0,8g |
| Guanidine carbonate | 7g |
| Huile minérale | 13,5g |
| Huile de paraffine | 16,5g |
| Alcool cétéarylique, PEG-75 lanoline, béhétrimonium méthosulfate | 10g |
| Agent alcalin | qsp pH=11 |
| Eau déminéralisée | qsp 100g |

| | |
|---|---|
| (*) Colorant fluorescent : 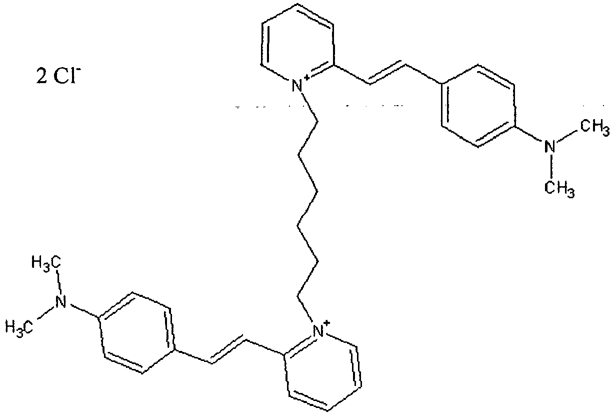 | |

Au moment de l'emploi, on mélange 30g de composition (i) et 235g de composition (ii), puis on applique le mélange obtenu sur cheveux frisés brun (hauteur de ton 2) pendant 20 minutes à température ambiante.

Après rinçage, lavage au shampooing, rinçage et séchage, les cheveux sont lissés au peigne et teints dans une nuance châtain foncé (hauteur de ton 3) à reflet cuivré mat.

### EXEMPLE 2

On a préparé les compositions suivantes (teneurs exprimées en grammes de matière active) :

**Composition (i)**

| | |
|---|---|
| Propylène glycol | 50,5g |
| Silice | 3g |
| Hydroxyde de calcium | 45g |
| Dioxyde de titane | 1,5g |

**Composition (ii)**

| | |
|---|---|
| Colorant fluorescent (**) | 10g |
| Eau déminéralisée | qsp 100g |

| | |
|---|---|
| (**) Colorant fluorescent : 2-(2-(4-diméthylamino-phényl)- vinyl)-1-éthyl-pyridinium éthyl sulfate: 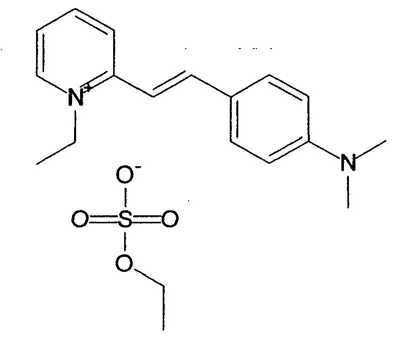 | |

**Composition (iii)**

| | |
|---|---|
| Guanidine carbonate | 7g |
| Huile minérale | 13,5g |
| Huile de paraffine | 16,5g |
| Alcool cétéarylique, PEG-75 lanoline, béhétrimonium méthosulfate | 10g |
| Agent alcalin | qsp pH=11 |
| Eau déminéralisée | qsp 100g |

Au moment de l'emploi, on mélange 30g de la composition (i), 12g de la composition (ii) et 235g de la composition (iii), puis on applique le mélange obtenu sur cheveux frisés brun (hauteur de ton 2) pendant 20 minutes à température ambiante.

Après rinçage, lavage au shampooing, rinçage et séchage, les cheveux sont lissés au peigne et teints dans une nuance avec une hauteur de ton 4 (châtain) et un reflet cuivré mat.

### EXEMPLE 3

On a préparé les compositions suivantes (teneurs exprimées en grammes de matière active) :

**Composition (i)**

| | |
|---|---|
| Mélange d'alcool cétylstéarylique, monostéarate de sorbitan oxyéthylène (20 OE) (60/40) | 8g |
| Huile de vaseline | 16g |
| Vaseline | 20g |
| Lanoline Oxyéthylénée (75 OE) | 0,75g |
| Chlorure de poly di-méthyl di-allylammonium dans l'eau à 40% non stabilisé | 1,25g |
| Méthosulfate de béhényl triméthyl ammonium à 25% dans l'alcool cétylstéarylique | 4g |
| Beurre de cacao | 0,01g |
| Beurre de karité protégé | 0,01g |
| Miel quaternisé (hydroxypropyl trimethyl ammonium) en solution aqueuse | 0,01g |
| Hydroxyde de sodium pur | 2g |
| Propylène glycol | 3g |
| Parfum | 0,2g |
| Eau déminéralisée | qsp 100g |

**Composition (ii)**

| | |
|---|---|
| Colorant fluorescent (***) | 10g |
| Alcool benzylique | qsp 100g |

| | |
|---|---|
| (***) Colorant fluorescent : 4-(4'-(N,N'-diméthylamino)phényl)- vinyl)-1-méthylpyridinium paratoluène sulfonate : 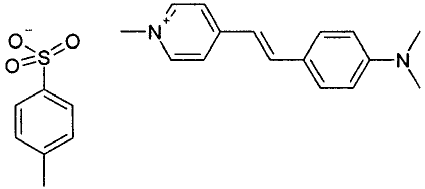 | |

Au moment de l'emploi, on mélange 100g de la composition (i) et 6g de la composition (ii), puis on applique le mélange obtenu sur cheveux blonds frisés pendant 20 minutes à température ambiante.

Après rinçage, lavage au shampooing, rinçage et séchage, les cheveux sont lissés au peigne et teints dans une nuance acajou très intense.

## Revendications

1. Composition de traitement des fibres kératiniques, comprenant dans un milieu cosmétiquement acceptable,
* un ou plusieurs colorants fluorescents choisis parmi les stilbazoliums ; les colorants fluorescents étant solubles dans ledit milieu,
* un ou plusieurs agents alcalins de type hydroxyde minéral ou organique en quantité telle que le pH de la composition est compris entre 10 et 14, et
* un ou plusieurs tensioactifs non ioniques, anioniques, cationiques ou amphotères.

2. Composition selon l'une quelconque des revendications précédentes, **caractérisée en ce que** le pH de la composition est compris entre 12 et 14.

3. Composition selon l'une quelconque des revendications précédentes, **caractérisée en ce que** l'agent alcalin de type hydroxyde minéral ou organique est choisi parmi les hydroxydes de métaux alcalins ou alcalino-terreux, de métaux de transition, en particulier des groupes IIIB, IVB, VB et VIB, de lanthanides ou d'actinides, les hydroxydes d'ammonium, de guanidine ou leurs mélanges.

4. Composition selon la revendication précédente, **caractérisée en ce que** l'agent alcalin est l'hydroxyde de guanidine.

5. Composition selon la revendication 3, **caractérisée en ce que** l'agent alcalin est l'hydroxyde de sodium.

6. Composition selon l'une quelconque des revendications précédentes, **caractérisée en ce qu'**elle comprend un ou plusieurs colorants fluorescents choisis parmi les composés suivants : 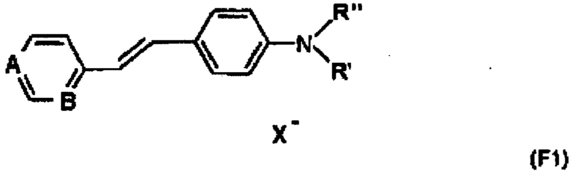
formule dans laquelle A et B désignent un groupement CH ou un groupement N⁺R, un seul de A ou B désignant un groupement CH ; R représente un radical alkyle linéaire, ramifié, ou cyclique comprenant 1 à 22 atomes de carbone, éventuellement substitué par au moins un groupement hydroxyle, par au moins un groupement alcoxy en C₁-C₈, linéaire ou ramifié, ou cycloalcoxy en C₁-C₆ et/ou par au moins un groupement cyano ;
R', R" indépendamment l'un de l'autre, représentent un atome d'hydrogène ; un radical alkyle, linéaire ou ramifié, comprenant 1 à 22 atomes de carbone, éventuellement substitué par un ou plusieurs radicaux hydroxyle ;
X représente un anion organique ou minéral.

7. Composition selon l'une quelconque des revendications précédentes, **caractérisée en ce qu'**elle comprend au moins une phase liquide inerte organique.

8. Composition selon la revendication précédente, **caractérisée en ce que** la ou les phases liquides inertes organiques sont choisies parmi par les polydécènes de formule C₁₀ₙH_{[(20n)+2]} dans laquelle n varie de 3 à 9, les esters tels que les esters d'alcools gras ou d'acides gras, les esters ou di-esters de sucres d'acides gras en C₁₂-C₂₄, ou les esters cycliques, les éthers cycliques, les huiles de silicone, les huiles minérales, les huiles végétales, et leurs mélanges.

9. Procédé de mise en forme, de coloration et/ou d'éclaircissement simultanés des fibres kératiniques, **caractérisé en ce que** l'on applique la composition tinctoriale, comprenant dans un milieu cosmétiquement acceptable, un ou plusieurs colorants fluorescents, tels que définis selon la revendication 1 ou 6 , solubles dans ledit milieu et un ou plusieurs agents alcalins de type hydroxyde minéral ou organique en quantité telle que le pH de la composition est compris entre 10 et 14, sur les fibres kératiniques pendant un temps suffisant pour développer la mise en forme, la coloration et/ou l'éclaircissement souhaités, puis on rince éventuellement les fibres kératiniques et on les lave au shampooing.

10. Procédé selon la revendication 9, **caractérisé en ce que** la composition est appliquée sur des cheveux présentant une hauteur de ton inférieure ou égale à 6 et de préférence inférieure ou égale à 4.

11. Dispositif à plusieurs compartiments, **caractérisé par le fait qu'**il comprend un premier compartiment (i) comprenant, dans un milieu cosmétiquement acceptable, un ou plusieurs agents alcalins de type hydroxyde minéral ou organique selon l'une des revendications 3 à 5, et un deuxième compartiment (ii) comprenant un ou plusieurs colorants fluorescents selon l'une des revendications 1 ou 6, le pH du mélange du contenu des différents compartiments étant compris entre 10 et 14.

12. Dispositif à plusieurs compartiments, **caractérisé par le fait qu'**il comporte un premier compartiment (i) comprenant du carbonate de guanidine et un ou plusieurs colorants fluorescents selon l'une des revendications 1 ou 6, et un deuxième compartiment (ii) comprenant un ou plusieurs agents alcalins de type hydroxyde d'un métal alcalin ou alcalino-terreux, le pH du mélange du contenu des différents compartiments étant compris entre 10 et 14.

13. Dispositif à plusieurs compartiments, **caractérisé par le fait qu'**il comporte un premier compartiment (i) comprenant du carbonate de guanidine, un deuxième compartiment (ii) comprenant un ou plusieurs agents alcalins de type hydroxyde d'un métal alcalin ou alcalino-terreux, et un troisième compartiment (iii) comprenant un ou plusieurs colorants fluorescents l'une des revendications 1 ou 6, le pH du mélange du contenu des différents compartiments étant compris entre 10 et 14.

## Patentansprüche

1. Zusammensetzung zur Behandlung von Keratinfasern, die in einem kosmetisch annehmbaren Medium Folgendes umfasst:
- einen oder mehrere in dem Medium lösliche fluoreszierende Farbstoffe, der/die aus Stilbazolium-Verbindungen ausgewählt ist/sind,
- ein oder mehrere alkalische Mittel des Typs eines anorganischen oder organischen Hydroxids in einer derartigen Menge, dass der pH der Zusammensetzung zwischen 10 und 14 beträgt, und
- ein oder mehrere nichtionische, anionische, kationische oder amphotere oberflächenaktive Mittel.

2. Zusammensetzung nach einem der vorhergehenden Ansprüche, **dadurch gekennzeichnet, dass** der pH der Zusammensetzung zwischen 12 und 14 beträgt.

3. Zusammensetzung nach einem der vorhergehenden Ansprüche, **dadurch gekennzeichnet, dass** das alkalische Mittel des Typs eines anorganischen oder organischen Hydroxids aus Hydroxiden von Alkalimetallen oder Erdalkalimetallen, Übergangsmetallen, insbesondere der Gruppen IIIB, IVB, VB und VIB, Lanthaniden oder Actiniden, Ammoniumhydroxid, Guanidiniumhydroxid oder deren Gemischen ausgewählt ist.

4. Zusammensetzung nach dem vorhergehenden Anspruch, **dadurch gekennzeichnet, dass** es sich bei dem alkalischen Mittel um Guanidiniumhydroxid handelt.

5. Zusammensetzung nach Anspruch 3, **dadurch gekennzeichnet, dass** es sich bei dem alkalischen Mittel um Natriumhydroxid handelt.

6. Zusammensetzung nach einem der vorhergehenden Ansprüche, **dadurch gekennzeichnet, dass** sie einen oder mehrere fluoreszierende Farbstoffe umfasst, der/die aus folgenden Verbindungen ausgewählt ist/sind: 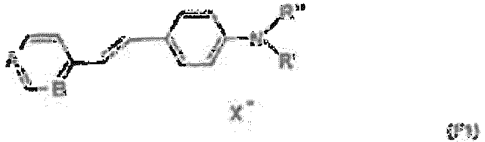
wobei A und B in der Formel für eine Gruppe CH oder eine Gruppe N⁺R stehen, wobei nur ein Rest aus A und B für eine Gruppe CH steht, R für einen geraden, verzweigten oder zyklischen Alkylrest steht, der 1 bis 22 Kohlenstoffatome umfasst und der gegebenenfalls mit mindestens einer Hydroxylgruppe, mit mindestens einer geraden oder verzweigten C₁-C₆-Alkoxygruppe oder C₁-C₆-Cycloalkoxygruppe und/oder mit mindestens einer Cyanogruppe substituiert ist,
R', R" unabhängig voneinander für ein Wasserstoffatom, einen geraden oder verzweigten Alkylrest, der 1 bis 22 Kohlenstoffatome umfasst und gegebenenfalls mit einem oder mehreren Hydroxylresten substituiert ist, stehen,
X für ein organisches oder anorganisches Anion steht.

7. Zusammensetzung nach einem der vorhergehenden Ansprüche, **dadurch gekennzeichnet, dass** sie mindestens eine inerte organische flüssige Phase umfasst.

8. Zusammensetzung nach dem vorhergehenden Anspruch, **dadurch gekennzeichnet, dass** die inerte(n) oxganische(n) flüssige(n) Phase(n) aus den Polydecenen der Formel C₁₀ₙH_{[(20n)+2]}, wobei n von 3 bis 9 variiert, Estern, wie Estern von Fettalkoholen oder Fettsäuren, Estern oder Diestern von Zuckern mit C₁₂-C₂₄-Fettsäuren oder zyklischen Estern, zyklischen Ethern, Silikonölen, Mineralölen, pflanzlichen Ölen und deren Gemischen ausgewählt wird/werden.

9. Verfahren zum gleichzeitigen Formen, Färben und/oder Aufhellen von Keratinfasern, **dadurch gekennzeichnet, dass** man die Färbezusammensetzung, die in einem kosmetisch annehmbaren Medium ein oder mehrere in dem Medium lösliche fluoreszierende Farbstoffe und ein oder mehrere alkalische Mittel des Typs eines anorganischen oder organischen Hydroxids in einer derartigen Menge, dass der pH der Zusammensetzung zwischen 10 und 14 beträgt, umfasst, auf die Keratinfasern für eine Dauer aufträgt, die ausreicht, dass sich die gewünschte Form, Färbung und/oder Aufhellung entwickelt, und dann gegebenenfalls die Keratinfasern spült und mit Shampoo wäscht.

10. Verfahren nach Anspruch 9, **dadurch gekennzeichnet, dass** die Zusammensetzung auf Haare aufgebracht wird, die eine Tonhöhe, wie in der Beschreibung definiert, von weniger als oder gleich 6 und vorzugsweise weniger als oder gleich 4 haben.

11. Vorrichtung mit mehreren Kompartimenten, **dadurch gekennzeichnet, dass** sie ein erstes Kompartiment (I) umfasst, das in einem kosmetisch annehmbaren Medium ein oder mehrere alkalische Mittel des Typs eines anorganischen oder organischen Hydroxids nach einem der Ansprüche 3 bis 5 umfasst, und ein zweites Kompartiment (II), das einen oder mehrere fluoreszierende Farbstoffe nach einem der Ansprüche 1 oder 6 umfasst, wobei der pH der Mischung des Inhalts der verschiedenen Kompartimente zwischen 10 und 14 beträgt.

12. Vorrichtung mit mehreren Kompartimenten, **dadurch gekennzeichnet, dass** sie ein erstes Kompartiment (I) enthält, das Guanidiniumcarbonat und einen oder mehrere fluoreszierende Farbstoffe nach einem der Ansprüche 1 oder 6 umfasst, und ein zweites Kompartiment (II), das ein oder mehrere alkalische Mittel des Typs eines Alkalimetall- oder Erdalkalimetallhydroxids umfasst, wobei der pH der Mischung des Inhalts der verschiedenen Kompartimente zwischen 10 und 14 beträgt.

13. Vorrichtung mit mehreren Kompartimenten, **dadurch gekennzeichnet, dass** sie ein erstes Kompartiment (I) enthält, das Guanidiniumcarbonat umfasst, ein zweites Kompartiment (II), das ein oder mehrere alkalische Mittel des Typs eines Alkalimetall- oder Erdalkalimetallhydroxids umfasst, und ein drittes Kompartiment (III), das einen oder mehrere fluoreszierende Farbstoffe nach einem der Ansprüche 1 oder 6 umfasst, wobei der pH der Mischung des Inhalts der verschiedenen Kompartimente zwischen 10 und 14 beträgt.

## Claims

1. Composition for treating keratin fibres, comprising in a cosmetically acceptable medium,
* one or more fluorescent dyes which are soluble in the said medium chosen from stilbazoliums,
* one or more inorganic or organic hydroxide type alkali agents in a quantity such that the pH of the composition is between 10 and 14, and
* one or more non-ionic, anionic, cationic or amphoteric surfactants.

2. Composition according to any one of the preceding claims, **characterized in that** the pH of the composition is between 12 and 14.

3. Composition according to any one of the preceding claims, **characterized in that** the inorganic or organic hydroxide type alkali agent is chosen from hydroxides of alkali or alkaline-earth metals, of transition metals, in particular of groups IIIB, IVB, VB and VIB, of lanthanides or of actinides, hydroxides of ammonium, of guanidine or mixtures thereof.

4. Composition according to the preceding claim, **characterized in that** the alkali agent is guanidine hydroxide.

5. Composition according to Claim 3, **characterized in that** the alkali agent is sodium hydroxide.

6. Composition according to any one of the preceding claims, **characterized in that** it comprises one or more fluorescent dyes chosen from the following compounds: 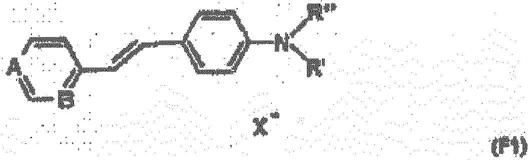
in which formula A and B denote a CH group or an N⁺R group, only one of A or B denoting a CH group; R represents a linear, branched, or cyclic alkyl radical comprising 1 to 22 carbon atoms, optionally substituted with at least one hydroxyl group, with at least one linear or branched C₁-C₆ alkoxy group or C₁-C₆ cycloalkoxy group and/or with at least one cyano group;
R', R", independently of each other, represent a hydrogen atom; a linear or branched alkyl radical comprising 1 to 22 carbon atoms, optionally substituted with one or more hydroxyl radicals;
X represents an organic or inorganic anion.

7. Composition according to any one of the preceding claims, **characterized in that** it comprises at least one organic inert liquid phase.

8. Composition according to the preceding claim, **characterized in that** the organic inert liquid phases are chosen from polydecenes of formula C₁₀ₙH_{[(20n)+2]} in which n varies from 3 to 9, esters such as fatty alcohol or fatty acid esters, sugar esters or diesters of C₁₂-C₂₄ fatty acids, or cyclic esters, cyclic ethers, silicone oils, mineral oils, vegetable oils, and mixtures thereof.

9. Method for the simultaneous styling, dyeing and/or lightening of keratin fibres, **characterized in that** the dye composition, comprising in a cosmetically acceptable medium, one or more fluorescent dyes which are soluble in the said medium and one or more inorganic or organic hydroxide type alkali agents in a quantity such that the pH of the composition is between 10 and 14, is applied to the keratin fibres for a sufficient time to develop the desired styling, dyeing and/or lightening, and then the keratin fibres are optionally rinsed and they are washed with shampoo.

10. Method according to Claim 9, **characterized in that** the composition is applied to hair having a tone height as defined in the description which is less than or equal to 6 and is preferably less than or equal to 4.

11. Multicompartment device, **characterized in that** it comprises a first compartment (i) comprising, in a cosmetically acceptable medium, one or more inorganic or organic hydroxide type alkali agents according to one of Claims 3 to 5, and a second compartment (ii) comprising one or more fluorescent dyes according to one of Claims 1 or 6, the pH of the mixture contained in the various compartments being between 10 and 14.

12. Multicompartment device, **characterized in that** it comprises a first compartment (i) comprising guanidine carbonate and one or more fluorescent dyes according to one of Claims 1 or 6, and a second compartment (ii) comprising one or more alkali or alkaline-earth metal hydroxide type alkali agents, the pH of the mixture of the content of the various compartments being between 10 and 14.

13. Multicompartment device, **characterized in that** it comprises a first compartment (i) comprising guanidine carbonate, a second compartment (ii) comprising one or more alkali or alkaline-earth metal hydroxide type alkali agents, and a third compartment (iii) comprising one or more fluorescent dyes according to one of Claims 1 or 6, the pH of the mixture of the content of the various compartments being between 10 and 14.
